# EUROPÄISCHE PATENTSCHRIFT

(11) **EP 2 173 387 B1**
(45) Veröffentlichungstag und Bekanntmachung des Hinweises auf die Patenterteilung: **04.07.2012**
(21) Anmeldenummer: 08785204.2
(22) Anmeldetag: 30.07.2008
(51) Int. Cl.: A61L 2/07, B65D 71/50, B65B 55/02, B65B 55/18

(54) **KOSTENGÜNSTIGES UND SICHERES STERILISATIONSVERFAHREN**
COST-EFFECTIVE AND SAFE STERILIZATION METHOD
PROCÉDÉ DE STÉRILISATION ÉCONOMIQUE ET SÛR

(30) Priorität: 03.08.2007 DE 102007036734
(43) Veröffentlichungstag der Anmeldung: 14.04.2010
(60) Teilanmeldung: 12002197.7
(73) Patentinhaber: Klosterfrau Berlin Gmbh, 12277 Berlin (DE)
(72) Erfinder: Venten, Ernst, verstorben (DE)
(74) Vertreter: Strehlke, Ingo Kurt
(86) Internationale Anmeldenummer: PCT/EP2008/006258
(87) Internationale Veröffentlichungsnummer: WO 2009/018948

(56) Entgegenhaltungen:
- WO-A-95/00180
- WO-A-03/074093
- WO-A-2007/000639
- CH-A- 223 434
- DE-A1-102005 045 504
- FR-A- 2 125 452

## Beschreibung

Die vorliegende Erfindung betrifft ein Verfahren zur Sterilisation, vorzugsweise mit Hilfe eines Dampf-Luft-Gemisches, mit den Merkmalen des Oberbegriffs von Anspruch 1.

Im medizinischen Bereich und darüber hinaus in vielen anderen, auch industriellen, Bereichen, in denen Sterilität ein Qualitätsmerkmal ist, werden eine Vielzahl von Sterilisationsverfahren angewendet. Typischerweise werden dabei Gegenstände in Sterilisationskammern eingebracht. Bei kostengünstigen Massenproduktionsverfahren ist es jedoch erforderlich, nicht nur effizient sondern auch kostengünstig eine Vielzahl von Gegenständen, insbesondere auch gleichartigen Gegenständen, zu sterilisieren.

Bei einem bekannten Sterilisationsverfahren (EP 0 703 793 B1) können Gegenstände, die in einer Faltschachtel verkauft werden sollen, zusammen mit dieser Faltschachtel sterilisiert werden. Die Gruppierung von Gegenständen in einer Faltschachtel ermöglicht die effiziente Handhabung einer großen Anzahl zu sterilisierender Gegenstände. Allerdings werden dafür gewisse Kompromisse eingegangen. Insbesondere wenn mit einem Dampf-Luft-Gemisch sterilisiert werden soll, ist die effiziente Heranführung des Dampf-Luft-Gemisches an die Gegenstände und die effiziente Umwälzung des Dampf-Luft-Gemisches wichtig. Im Übrigen ist in aller Regel eine Sterilisation der äußeren Umverpackung, also etwa der Faltschachtel, nicht erforderlich, da bei späteren Handhabungsschritten diese Umverpackung ohnehin nicht steril gehalten werden kann.

In einem weiteren Aspekt, der gerade für die Bereitstellung steriler medizinischer Gegenstände eine Rolle spielt, ist es wichtig, dass das Erscheinungsbild der äußeren Verpackung durch das Sterilisationsverfahren nicht leidet. Wenn aber beispielsweise Faltschachteln einer typischen Dampf-Luft-Sterilisation ausgesetzt sind, weisen nicht selten einzelne Faltschachteln Verfärbungen oder Wasserflecken auf Sofern die Faltschachteln auf Gestellen oder dergleichen angeordnet sind, entstehen während eines Dampf-Luft-Sterilisationsprozesses auch leicht Druckstellen. Häufig sind solche Druckstellen, wie auch die Wasserflecken, dauerhaft sichtbar.

Wünschenswert wäre es also, den offenbarten Prozess seiner Qualität nach zu verbessern, ohne aber die Kosteneffizienz leiden zu lassen. Idealerweise wird eine Qualitätsverbesserung bei zugleich höherer Wirtschaftlichkeit erreicht. Insbesondere wäre es auch wünschenswert, einen Prozess zu gestalten, der mit handelsüblichen Einrichtungen und Apparaten auskommt, und der möglicherweise auf einer solchen Sterilisationsanlage ausgeführt werden kann, wie sie bislang für die Sterilisation mit Faltschachteln eingesetzt wird.

Das bekannte Verfahren zur Sterilisation, von dem die Erfindung ausgeht (CH-A-223 434), kommt den zuvor beschriebenen Zielen schon etwas näher, weil hier auf eine Faltschachtel bei der Sterilisation verzichtet wird. Bei dem dortigen Verfahren geht es ebenfalls um die Sterilisation von Gegenständen, die untereinander gleichartig und länglich sind und je eine Achse L aufweisen, die die längste der Hauptachsen des Gegenstands ist und die mehr als doppelt so lang ist wie die anderen Hauptachsen des Gegenstands. Die Gegenstände einer Gruppe werden hier als Bündel in einen Sterilisationsraum eingebracht, darin sterilisiert und wieder aus dem Sterilisationsraum entnommen.

Ausgehend von dem zuvor beschriebenen Stand der Technik liegt der Lehre das Problem zugrunde, das bekannte Verfahren mit einfachen Mitteln zeit- und kosteneffizienter zu gestalten und damit das Verfahren in vorteilhafter Weise weiterzubilden.

Das zuvor aufgezeigte Problem wird bei einem Verfahren mit den Merkmalen des Oberbegriffs von Anspruch 1 durch die Merkmale des kennzeichnenden Teils von Anspruch 1 gelöst.

Im Rahmen der vorliegenden Erfindung wird vorgeschlagen, ein Umhüllungsmittel im Sterilisationsverfahren einzusetzen. Dieses Umhüllungsmittel ist so auszulegen, dass es eine Gruppe von Gegenständen teilweise, aber nicht vollständig umhüllt. In Vorbereitung ihrer Sterilisation können die Gegenstände in günstiger Weise in Gruppen angeordnet werden. Je eine Gruppe von Gegenständen wird dann mit dem Umhüllungsmittel umhüllt. Da diese Umhüllung die Gegenstände nur teilweise, aber nicht vollständig bedeckt, wird die Sterilisation begünstigt, insbesondere kann an den unverhüllten Seiten ein Dampf-Luft-Gemisch eintreten und auch austreten. Dabei kann der Eintritt und der Austritt an gegenüberliegenden Seiten der Gruppe von Gegenständen sein. Die Umhüllung ihrerseits stellt sicher, dass die Anordnung der Gruppe von Gegenständen erhalten bleibt. Diese Anordnung kann bezogen auf die Sterilisation und/oder auf die spätere Verpackung optimiert sein.

Die im Folgenden beschriebenen Vorrichtungen Können zur Durchführung des erfindungsgemäß Verfahren verwendet werden, wobei diese Vorrichtungen nicht gemäß der Erfindung sind.

Das Verfahren eignet sich besonders gut auch zur Sterilisation eher länglicher Gegenstände. Diese Gegenstände können dann entlang ihrer längsten Länge parallel angeordnet werden, und zwar so, dass zwischen ihnen Durchströmkanäle für ein Gas-Luft-Gemisch verbleiben. Die Umhüllung wird dann so angebracht, dass ein Bündel von Gegenständen entsteht und das Umhüllungsmaterial dem Rand einer Fläche folgt (oder den Rändern einer Vielzahl paralleler Flächen), die im Wesentlichen senkrecht zu den Längsachsen der Gegenstände ist. Dies ermöglicht eine gute mechanische Stabilisierung der Gruppe und erhält zugleich günstige Durchströmkanäle für den Sterilisationsprozess.

Insbesondere ist das Verfahren auch für unregelmäßig geformte Gegenstände geeignet, die über konkav-konvexe Konturen verfügen, welche sich aber so anordnen lassen, dass eine (konvexe) Ausbuchtung des einen Gegenstandes in eine (konkave) Einbuchtung des anderen Gegenstandes eingreift. Gerade solche Gegenstände lassen sich raumsparend in Gruppen anordnen, und eventuell in mindestens einem Querschnitt oder vollständig formschlüssig anordnen, was für den Sterilisationsprozess erhebliche Kostenvorteile bringt. Durch die konkav-konvexe Form der Gegenstände ergeben sich jedoch gebogene Durchströmkanäle. Wenn die Einlass- oder Auslassöffnungen solche Durchströmkanäle durch Verpackungsmaterial oder dergleichen blockiert werden, ist eine effiziente Sterilisierung erschwert. Eine Umhüllung von Seiten, an denen solche Durchströmkanäle nur in kleiner Zahl, oder mit kleinem Querschnitt oder gar nicht vorhanden sind, ist jedoch weniger problematisch.

In Weiterbildung der Erfindung kann die Umhüllung auch dazu dienen, eine erfolgreiche Sterilisation anzuzeigen. Dies bietet sich insbesondere deshalb an, weil die Umhüllung annähernd identischen Sterilisationsbedingungen ausgesetzt ist wie die umhüllte Gruppe von Gegenständen. Zu diesem Zweck kann ein Indikator an der Umhüllung angebracht werden. Ein geeigneter Indikator ist ein Farbindikator, der bei einem definierten "F0-Wert" umschlägt. In einer bevorzugten Ausführung ist der Farbindikator automatisch lesbar.

In Weiterbildung der Erfindung kann die Umhüllung auch als Originalitätsverschluss dienen. Ein solcher Verschluss soll einem Käufer oder Benutzer der Gegenstände anzeigen, dass sie sich noch im Originalzustand befinden, das heißt in dem Zustand wie hergestellt, insbesondere auch wie sterilisiert.

In Weiterbildung der Erfindung kann die Umhüllung auch dazu dienen, eine Transportmöglichkeit für die manuelle oder maschinelle Handhabung einer Gruppe von Gegenständen anzubieten. Als Transportmöglichkeit kommt das Greifen der Umhüllung an beliebiger Stelle in Frage, vorzugsweise ist die Umhüllung mit einer Greifvorrichtung, z.B. einer Griffschlaufe versehen.

Diese und noch weitere Merkmale der Erfindung gehen aus den Ansprüchen hervor, aber auch aus der nachfolgenden Beschreibung und den zugehörigen Zeichnungen. Die Erfindung wird anhand folgender Zeichnungen näher erläutert:
FIG.1 - schematischen Ablauf des Verfahrens. Dabei steht "G" für das Gruppieren von Gegenständen, "S" steht für das Sterilisieren von Gegenständen und "V" steht für das Verpacken von einer Gruppe von Gegenständen.
FIG.2 - zeigt Gegenstände, die zu einer Gruppe angeordnet sind.
FIG.3 - zeigt einen Längsschnitt entlang der Linie III-III aus der FIG.2.
FIG.4 - zeigt eine Gruppe von Gegenständen zusammen mit einer Umhüllung.
FIG.5 - zeigt einen Längsschnitt entlang der Linie V- V durch die Gruppe von Gegenständen aus FIG.4.
FIG. 5A -zeigt einen Längsschnitt nach Art der FIG. 5, jedoch für eine Ausführungsform, bei der die Umhüllung zusätzlich eine Transportmöglichkeit in Form einer Griffschlaufe aufweist.
FIG. 5B -zeigt einen Längsschnitt nach Art der FIG. 5, jedoch für eine Ausführungsform, bei der die Umhüllung als Stülpboden ausgeführt ist.
FIG.6 - zeigt die gleiche Gruppe von Gegenständen wie FIG.2 und FIG.4 in der Draufsicht und nach dem Einbringen in ein Verpackungsmittel.
FIG.7 - zeigt eine Ansicht entlang der Linie VII-VII aus FIG.6.

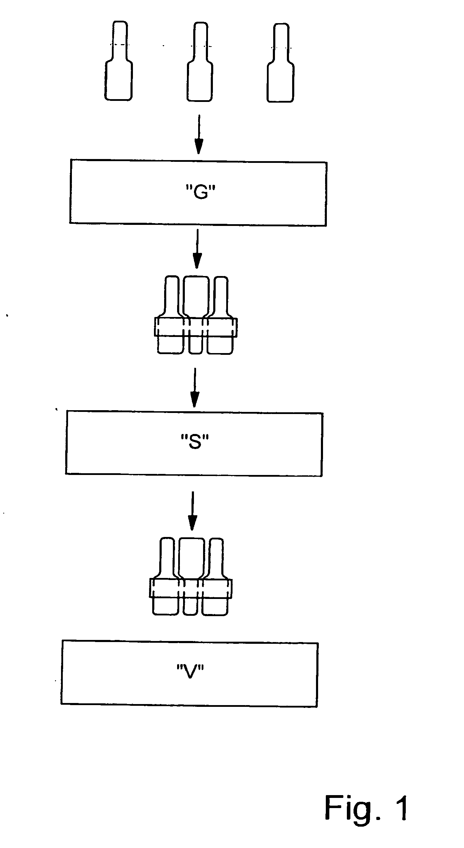

In der Fig. 1 sind die Grundzüge des Verfahrens schematisch dargestellt. Gegenstände (10) werden zunächst zu geeigneten Gruppen (20) zusammengefasst. Dies kann sowohl von Hand, als auch maschinell, als auch automatisch geschehen. Die so gebildete mindestens eine Gruppe (20) von Gegenständen (10) werden im nächsten Schritt mit einer Umhüllung (30) versehen. Diese Umhüllung (30) bedeckt die Gruppe (20) von Gegenständen (10) teilweise, jedoch nicht vollständig. Die Gruppe (20) von Gegenständen (10) wird im nächsten Schritt einschließlich ihrer Umhüllung (30) in einen Sterilisationsraum eingebracht. Erfindungsgemäß können auch zwei oder drei oder eine Vielzahl, zum Beispiel mehr als hundert, oder mehr als zweihundert, oder mehr als dreihundert Gruppen (20) von Gegenständen (10) in den Sterilisationsraum eingebracht werden. Die Gegenstände (10) werden dann dort in geeigneter Weise sterilisiert. Die Sterilisationsbedingungen können dabei auch von der Natur der Gegenstände (10), aber auch von der Art ihrer Anordnung in Gruppen (20), von der Anzahl der Gruppen (20), und von der Art der Umhüllung (30) abhängig sein. Die Gegenstände (10) werden nach Abschluss der Sterilisation, immer noch in der einmal gewählten Gruppenanordnung, dem Sterilisationsraum entnommen. Im nächsten Schritt wird je eine Gruppe (20) von Gegenständen (10) einschließlich (oder zumindest mit Hilfe) ihrer Umhüllung (30) in ein Verpackungsmittel (40) eingebracht. Erfindungsgemäß ist dieses Verpackungsmittel (40) zur vollständigen Umhüllung (30) der Gruppe (20) von Gegenständen (10) geeignet.

In einer alternativen Ausführungsform der Erfindung, wird die vollständige Umhüllung (30) der Gruppe (20) von Gegenständen (10) durch das Verpackungsmittel (40) erst im Zusammenspiel mit der Umhüllung (30) der Gruppe (20) von Gegenständen (10) erreicht. Beispielsweise könnte die Umhüllung (30) eine gut schließende Banderole bilden, die bereits eine großflächige Umhüllung (30) der Gegenstände (10) gestattet, und das Verpackungsmittel (40) ist etwa in Form von Kappen angebracht, welches beispielsweise die Stirnseiten einer Gruppe (20) von Gegenständen (10) aufnimmt.

In einer bevorzugten Ausführungsform, der Erfindung jedoch, ist das Verpackungsmittel (40) auch ohne die Umhüllung (30) zur vollständigen Umhüllung (30) der Gruppe (20) von Gegenständen (10) geeignet. Dies erlaubt es, dass die Umhüllung (30) nach dem Einbringen der Gruppe (20) von Gegenständen (10) in das Verpackungsmittel (40) entfernt wird. Alternativ jedoch kann die Gruppe (20) von Gegenständen (10) einschließlich ihrer Umhüllung (30) vom Verpackungsmittel (40) aufgenommen werden und die Umhüllung (30) verbleibt im Verpackungsmittel (40). An den Schritt des Einbringens der Gruppe (20) von Gegenständen (10) in das Verpackungsmittel (40) schließt sich vorzugsweise das Verschließen des Verpackungsmittels (40) an.

Vorzugsweise ist die Gruppe (20) von Gegenständen (10) die umhüllt wird, identisch mit der Gruppe (20) von Gegenständen (10), die im Sterilisationsraum sterilisiert wird. Es findet also keine Umgruppierung der Gegenstände (10) statt. Weiter ist vorzugsweise diese Gruppe (20) von Gegenständen (10) auch identisch mit der Gruppe (20) von Gegenständen (10), die in das Verpackungsmittel (40) eingebracht wird. Erfindungsgemäß kommt es jedoch in Betracht, dass mehr als eine Gruppe (20) von Gegenständen (10) in das Verpackungsmittel (40) eingebracht wird. Vorzugsweise werden also entweder eine oder zwei oder drei oder vier Gruppen (20) von Gegenständen (10) jeweils in ein Verpackungsmittel (40) eingebracht.

In einer bevorzugten Ausführungsform der Erfindung werden die Gruppen (20) von Gegenständen (10) auf geeigneten Gestellen oder in so genannten Sterilisationskörben eingeordnet und mit deren Hilfe in den Sterilisationsraum eingebracht, vorzugsweise auch mit Hilfe der Gestelle oder Körbe dort sterilisiert und anschließend dem Sterilisationsraum wieder entnommen.

Die vorliegende Erfindung bezieht sich auch auf ein Verfahren zur Sterilisation von Gegenständen (10). Vorteilhafte und hierin dargelegte Ausgestaltungen des Verfahrens werden erfindungsgemäß mit angepassten Vorrichtungen der Anlage ausgeführt. Es ist erfindungsgemäß einzelne oder alle Vorrichtungen der Anlage auf maschinellen, am besten automatischen Betrieb hin auszulegen. Die erfindungsgemäße Anlage umfasst zumindest eine automatische Vorrichtung zur Anordnung von Gegenständen (10) in Gruppen (20). Sie umfasst ferner eine automatische Vorrichtung um je eine Gruppe (20) von Gegenständen (10) eine die Gruppe (20) teilweise, aber nicht vollständig bedeckenden Umhüllung (30) versehen. Diese Anlage umfasst ferner Vorrichtungen, um mindestens eine Gruppe (20) von Gegenständen (10) einschließlich der Umhüllung (30) in den Sterilisationsraum einzubringen. Diese Beschickungsvorrichtung kann Sterilisationsgestelle oder Sterilisationskörbe umfassen. Die Beschickungsvorrichtung können die vollautomatische Einbringung der Gegenstände (10) in den Sterilisationsraum vorsehen oder die benutzerunterstützte oder vollständig manuelle Einbringung. Die Anlage umfasst ferner eine Sterilisationskammer. Ferner umfasst die erfindungsgemäße Anlage Vorrichtungen zur Entnahme der Gegenstände (10) aus dem Sterilisationsraum. Diese Vorrichtungen können identisch mit denjenigen der Einbringung der Gegenstände (10) in den Sterilisationsraum sein. Die Beschickungs- und die Entnahmevorrichtung können auch teilweise identisch sein, d.h. gemeinsame Teile umfassen, beispielsweise Sterilisationsgestelle und -körbe. Ferner umfasst die erfindungsgemäße Anlage automatische Vorrichtungen, mit denen je eine Gruppe (20) von Gegenständen (10) einschließlich ihrer Umhüllung (30) in ein Verpackungsmittel (40) eingebracht werden können. Vorzugsweise umfasst die erfindungsgemäße Anlage eine Sterilisationskammer, die für die Sterilisierung mit einem Dampf-Luft-Gemisch ausgelegt ist. Es ist weiterhin erfindungsgemäß, dass die Anlage mit all solchen Vorrichtungen ausgestattet werden kann, die es erlauben, das hierin beschriebene Verfahren, einschließlich all seiner bevorzugten Ausgestaltungen durchzuführen.

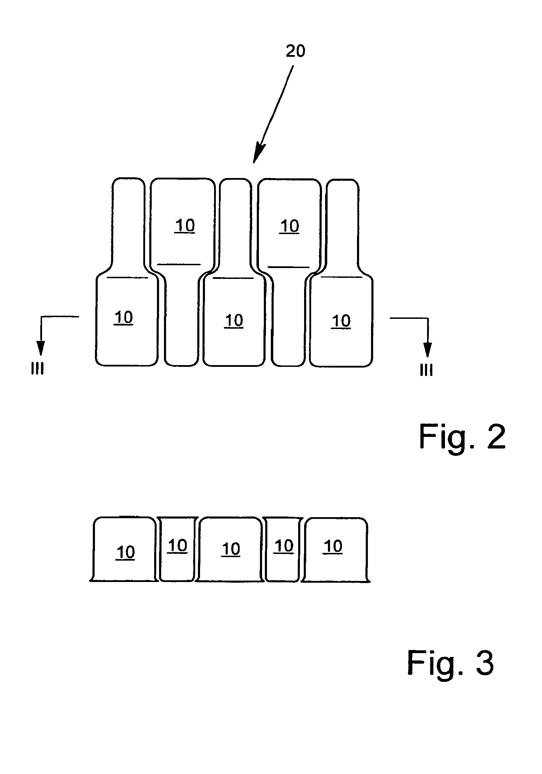

Fig. 2 zeigt in einer Gruppe (20) angeordnete Gegenstände (10). Das vorgestellte Verfahren ist im Prinzip für beliebige Gegenstände (10) geeignet. Es können sowohl gleichartige als auch verschiedenartige Gegenstände (10) gemeinsam sterilisiert werden und in Gruppen (20) angeordnet werden. Vorzugsweise werden jedoch gleichartige Gegenstände (10) in Gruppen (20) angeordnet. Diese Anordnung kann entweder optimiert werden für den Sterilisationsschritt und/oder für die spätere Verpackung. In der Regel ist aber schon für das Einbringen in den Sterilisationsraum eine Raum sparende Anordnung vorteilhaft.

Das Verfahren ist zur Sterilisation von länglichen Gegenständen (10) geeignet. Ein solcher Gegenstand kann zweckmäßig durch seine Hauptachsen, dass heißt seine Hauptträgheitsachsen beschrieben werden. Wenn der Gegenstand Symmetrieachsen aufweist, sind diese Symmetrieachsen auch Hauptachsen. Entlang einer Hauptachse wird der Gegenstand die größte Ausdehnung haben. Hierin wird diese Achse als Achse L bezeichnet. Beispielsweise wäre für eine (rotationssymmetrische) Flasche, etwa eine typische Weinflasche, die Achse L die Längsachse, die den Mittelpunkt der Flaschenöffnung mit dem Mittelpunkt des Bodens verbindet.

Erfindungsgemäß werden längliche Gegenstände (10) vorzugsweise so gruppiert, dass ihre Achsen L parallel ausgerichtet sind. Wie die Fig. 2 zeigt, müssen dabei jedoch die Gegenstände (10) nicht alle in die gleiche Richtung ausgelegt werden. Insbesondere wenn die Gegenstände (10) Ausbuchtungen und Einbuchtungen aufweisen, ist es häufig vorteilhaft die Gegenstände (10) so auszurichten, dass diese ineinander eingreifen können. In einer bevorzugten Ausführungsform der Erfindung werden längliche Gegenstände (10) mit gegensinning orientierten Achsen angeordnet. Eine solche Anordnung ist auch in Fig. 2 gezeigt.

Werden die Gegenstände (10) hinreichend beabstandet, führt dies dazu, dass die Gruppe (20) von Gegenständen (10) bei der Sterilisation generell parallel zu den Achsen L von einem Dampf-Luft-Gemisch durchströmt werden kann. Die Strömungskanäle verlaufen allerdings dabei nicht genau parallel zu den Achsen L, sondern sind vielmehr gekrümmt. Um angesichts der Krümmung der Durchströmkanäle die Durchströmung der Gruppe (20) von Gegenständen (10), und damit Ihre Sterilisation, nicht weiter zu behindern, ist es vorteilhaft, wenn die Stirnflächen der Gruppe (20) (das sind die Flächen, die senkrecht auf den Achsen L stehen) nicht verdeckt werden. Daher wird erfindungsgemäß die Gruppe (20) der Gegenstände (10) so umhüllt, dass die Umhüllung (30) die Stirnflächen der Gruppe (20) nicht bedeckt. Vorzugsweise beugt dazu die Umhüllung (30) im Wesentlichen den Rand einer oder mehrerer Flächen, die senkrecht zu den Achsen L steht/stehen.

Das Sterilisationsverfahren eignet sich durchaus auch zur Sterilisation von Gegenständen (10), die nicht symmetrisch zur Achse L sind. Dies zeigt auch Fig. 3, welche eine Querschnittsansicht der Gruppe (20) der Gegenstände (10) aus Fig. 2 zeigt. Aus der Fig. 3 ist jedoch auch offenkundig, dass die Gruppe (20) von Gegenständen (10) raumsparend angeordnet ist und Ausbuchtungen in Einbuchtungen eingreifen, so dass vollständige oder annähernde Formschlüssigkeit erreicht wird.

Im Sinne der vorliegenden Erfindung wurde erkannt, dass gerade bei einer räumlich optimierten und kompakten Bildung von Gruppen (20) von Gegenständen (10), die gute Durchströmbarkeit wesentlich ist. Ebenfalls kann es wesentlich sein, durch adäquate mechanische Stabilisierung überhaupt eine räumlich optimale Anordnung von Gegenständen (10) zu erzielen und während des Sterilisationsprozesses und Verpackungsprozesses zu - erhalten. Das Verfahren hat besondere Vorteile, wenn die zu sterilisierenden Gegenstände (10) länglich sind und die Achse L mehr als doppelt so lang ist wie die anderen Hauptträgheitsachsen.

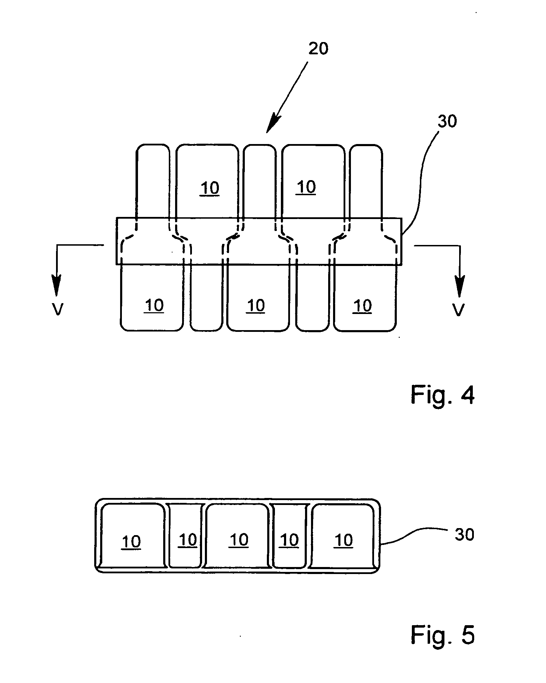

Fig. 4 zeigt in der Draufsicht die vorteilhafte Anbringung einer Umhüllung (30) für die Gruppe (20) von Gegenständen (10), die bereits aus Fig. 2 bekannt ist.

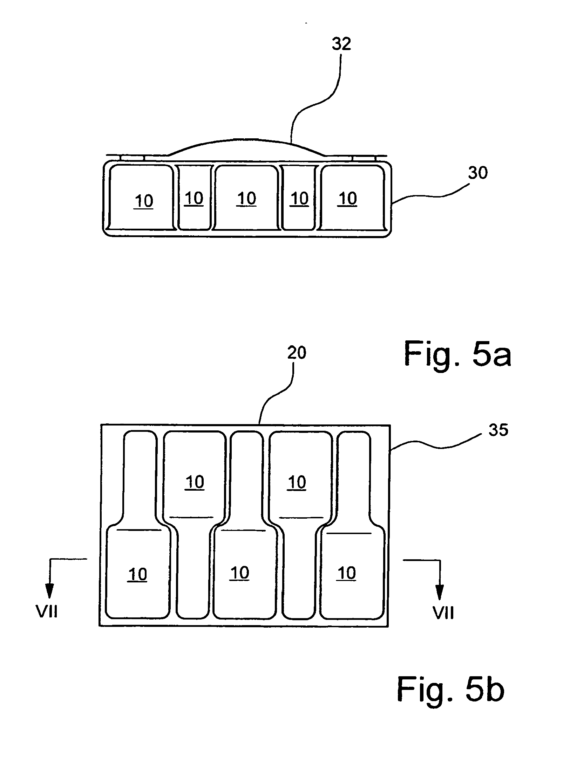

Fig. 5 zeigt eine entsprechende Querschnittsansicht. Wie eng die Umhüllung (30) die Gegenstände (10) umschließen soll, hängt wiederum von der mechanischen Empfindlichkeit der Gegenstände (10) ab, aber auch von strömungstechnischen Erwägungen zum Sterilisationsprozess.

Erfindungsgemäß kann die Umhüllung (30) auch mit einer Transporthilfe oder Greifvorrichtung ausgestattet sein, z.B. einer Griffschlaufe, einem Zapfen oder einer Zapfenaufnahme, einem Magneten oder einem Klettmaterial. Solche Vorrichtungen geben den Umhüllung (30) den Zusatznutzen, dass die Gruppe (20) von Gegenständen (10) leichter transportiert werden und leichter in das Verpackungsmittel (40) eingebracht werden kann. Eine Umhüllung (30) mit Griffschlaufe (32) ist in Fig. 5A gezeigt.

Fig. 5B zeigt ein alternatives erfindungsgemäßes Umhüllungskonzept. Die Umhüllung kann ein Stülpboden (35) sein. Zum Einbringen der Gegenstände in das Verpackungsmittel kann der Stülpboden (35) umgestülpt werden.

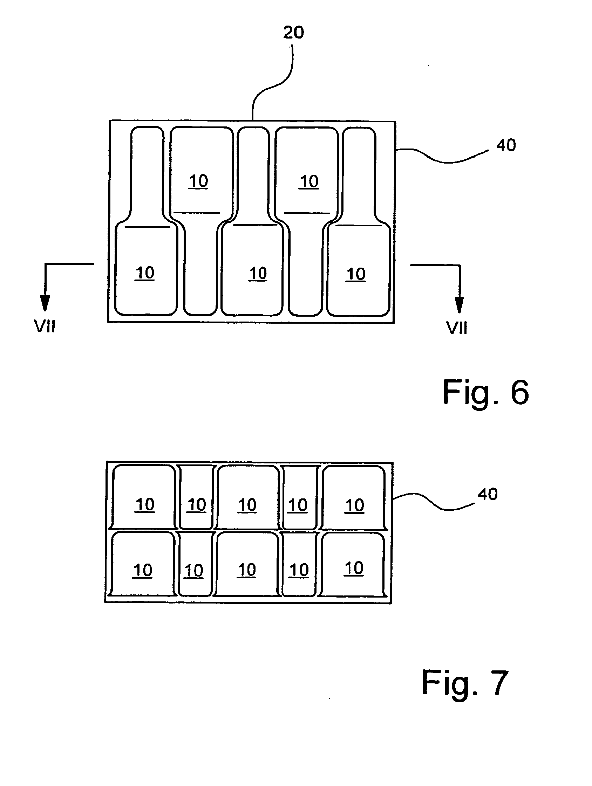

Fig. 6 zeigt in der Draufsicht eine Gruppe (20) von Gegenständen (10), nämlich diejenige die aus Fig. 2 bekannt ist, nachdem sie in ein Verpackungsmittel (40) eingebracht wurde. Erfindungsgemäß wird die Gruppe (20) von Gegenständen (10) samt der Umhüllung (30) in das Verpackungsmittel (40) eingebracht. Das Verpackungsmittel (40) sollte gerade so viel Platz bieten, dass eine oder mehrere Gruppen (20) von Gegenständen (10) einschließlich der Umhüllungen (30) gut aufgenommen werden können. Das so dimensionierte Verpackungsmittel (40) kann die Funktion der mechanischen Stabilisierung der Gruppe (20) von Gegenständen (10) übernehmen. Daher kann die Umhüllung (30) nach dem Einbringen in das Verpackungsmittel (40) entfernt werden.

Alternativ jedoch kann die Umhüllung (30) im Verpackungsmittel (40) verbleiben, das heißt, das Verpackungsmittel (40) wird ohne Entfernen der Umhüllung (30) verschlossen.

Fig. 7 zeigt zwei Gruppen (20) von Gegenständen (10), die gemeinsam in ein Verpackungsmittel (40) eingebracht sind. Erfindungsgemäß kann eine einzelne Gruppe (20) von Gegenständen (10) in das Verpackungsmittel (40) eingebracht werden, vorzugsweise werden aber auch zwei oder drei oder vier oder mehrere oder noch mehr Gruppen (20) von Gegenständen (10) in ein Verpackungsmittel (40) eingebracht. Wie es die Fig. 7 zeigt, müssen die Gruppen (20) von Gegenständen (10) dabei nicht in sich identisch angeordnet sein. Erfindungsgemäß können verschieden angeordnete Gruppen (20) gleicher Gegenstände (10) in das Verpackungsmittel (40) eingebracht werden oder auch Gruppen (20) verschiedener Gegenstände (10). Außerdem können Gruppen (20) gleicher Gegenstände (10) verschieden orientiert werden, beispielsweise kann vor dem Einbringen in das Verpackungsmittel (40) eine Gruppe (20) einmal um 90° oder 180 ° um eine ihrer Achsen, vorzugsweise Hauptachsen, gedreht werden. Eine solche Anordnung kann zur mechanischen Stabilität der Verpackung einschließlich der Gegenstände (10) beitragen.

An der Fig. 7 wird auch erkennbar, dass die Umhüllung (30) als Originalitätsverschluss gestaltet werden kann. Dazu ist die Umhüllung (30) so auszulegen, dass sie nicht unbemerkt geöffnet werden kann und die Gegenstände (10) nicht unbemerkt aus der Umhüllung (30) entnommen werden können. Ferner ist die Umhüllung (30) vorzugsweise mit dem Verpackungsmittel (40) so zu verbinden, dass eine Entnahme der Umhüllung (30) aus dem Verpackungsmittel (40) sichtbar wird. Vorzugsweise wird die Umhüllung (30) mit einer Kennnummer und einem Haltbarkeitsdatum für die umhüllten Gegenstände (10) (z.B. Ch.-B.-Angabe) versehen. Eine entsprechende Kennung kann auch für das Verpackungsmittel (40) vorgesehen werden.

Die vorliegende Erfindung kann im Zusammenhang mit verschiedenen bekannten Sterilisationsverfahren eingesetzt werde, die zum Sterilisieren von Gruppen (20) von Gegenständen (10) geeignet sind. Besonders vorteilhaft wird es zur Sterilisation mit einem Dampf-Luft-Gemisch eingesetzt und bei Sterilisationstemperaturen im Bereicht von 120 bis 140 ° C. Besonders vorteilhaft wirkt sich die Erfindung bei Verfahren mit hoher Dampf-Luft-Umwälzgeschwindigkeit und hoher Evakuierungsgeschwindigkeit aus. Das hierin beschriebene und beanspruchte Verfahren zur Sterilisation von Gegenständen kann sehr vorteilhaft bei Verfahren mit Dampf-Luft-Umwälzgeschwingdigkeiten von mehr als 3 Metern pro Sekunde oder mehr als 10 Metern pro Sekunde (m/s) eingesetzt werden, besonders Dampf-Lun-Umwälzgeschwingdigkeiten im Bereich von 3 m/s bis 300 m/s, vorzugsweise 10 m/s bis 100 m/s, besonders vorzugsweise 20 m/s bis 50 m/s.

Vorteilhaft kann die vorliegende Erfindung bei Dampf-Luft-Sterilisationen eingesetzt werden, bei denen die Sterilisationskammer von Fugen oder ähnlichen Kältebrücken durchbrochene Wandflächen aufweist. Entsprechende Fugen können Türfugen sein. Es hat sich gezeigt, dass bei solchen Kammern besonders leicht Wasserflecken auftreten.

Im Sinne der vorliegenden Erfindung kann ein Gegenstand durchaus aus vielen Einzelteilen bestehen. Insbesondere kann ein Gegenstand bereits eine individuelle Verpackung aufweisen. Erfindungsgemäß kommen als zu sterilisierende Gegenstände insbesondere Einwegspritzen, auch gefüllte Einwegspitzen, in Frage. Solche Spritzen können individuell zum Beispiel durch eine Blisterverpackung verpackt sein. Solche Blisterverpackungen umfassen eine gewölbte Haube und eine die Haube auf einer Seite verschließende mit ihr verschweißte Schicht. Diese Schicht kann manuell abgezogen werden, um den Inhalt zu entnehmen. Ein Gegenstand im Sinne der Anmeldung ist also beispielsweise, aber auch vorzugsweise, eine gefüllte Einwegspritze in einer Blisterverpackung.

Für die Umhüllung kommt eine Vielzahl an Materialien und Formen in Betracht. Wichtig ist zum einen, dass die Umhüllung eine ausreichende mechanische Stabilität der Gruppe von Gegenständen bewahrt. Insbesondere sollte sie das leichte Handhaben der Gruppe von Gegenständen beim Einbringen in die Verpackung unterstützen. Ferner ist wichtig, dass die Umhüllung die Sterilisation der Gegenstände nur geringfügig beeinträchtigt. Insbesondere sollte die Umhüllung die Durchströmung der Gegenstände mit einem Dampf-Luft-Gemisch nur minimal beeinträchtigen, aber auch die Evakuierung nur minimal beeinträchtigen. Die Umhüllung kann insbesondere als breites oder schmales Band ausgeführt sein (evtl. auch als Litze oder dgl.). Vorzugsweise ist die Umhüllung reißfest, wasserfest und temperaturbeständig bis mindestens 140°C.

Die Umhüllung selbst sollte im Sterilisationsprozess keinen Schaden leiden und durch denselben Prozess wie die Gegenstände sterilisierbar sein. Die Umhüllung sollte auch so bemessen sein, dass keine Druckspuren an den Gegenständen entstehen. Die Umhüllung kann aus Pappe, Papier oder einem Verbundmaterial hergestellt werden, vorzugsweise aber aus Kunststoff, d.h. polyolefinem Kunststoff, beispielsweise aus Kunststofffolie, insbesondere aus einer Polypropylenfolie. Vorzugsweise ist die Umhüllung aus einem Material, das verschieden von den umhüllten Materialien der Gegenstände ist. Dies vermeidet Wechselwirkungen (z.B. Verkleben oder Verschmelzen) der Materialien während des Sterilisationsverfahrens.

Vorzugsweise weist die Umhüllung auch einen Indikator auf, der die erfolgreiche Sterilisation anzeigt. Wird eine solche Umhüllung verwendet, ist es im Sinne der Erfindung vorzuziehen, wenn diese Umhüllung vor Verschließen des Verpackungsmittels entfernt wird und in weiteren Sterilisationsprozessen wieder verwendet wird.

## Patentansprüche

1. Verfahren zur Sterilisation, vorzugsweise mit Hilfe eines Dampf-Luft-Gemisches, von Gegenständen (10), die untereinander gleichartig und länglich sind und je eine Achse L aufweisen, welche die längste der Hauptachsen des Gegenstandes (10) ist und welche mehr als doppelt so lang wie die anderen Hauptachsen des Gegenstandes (10) ist,
wobei das Verfahren folgende Schritte umfasst:
a) die Gegenstände (10) werden in mindestens einer Gruppe (20) so angeordnet, dass ihre Hauptachsen L parallel zueinander ausgerichtet sind und die Gegenstände (10) der Gruppe (20) ein Bündel bilden,
b) die so gebildete Gruppe (20) von Gegenständen (10) wird als Bündel in einen Sterilisationsraum eingebracht, in dem Sterilisationsraum sterilisiert, dem Sterilisationsraum entnommen und in ein Verpackungsmittel (40) eingebracht, welches zur vollständigen Umhüllung zumindest dieser Gruppe (20) von Gegenständen (10) geeignet ist,
**dadurch gekennzeichnet,**
**dass** die Gruppe (20) von Gegenständen (10) mit einer sie teilweise, aber nicht vollständig bedeckenden Umhüllung (30) versehen wird, die das Bündel von Gegenständen (10) schafft, im Wesentlichen dem Rand einer Fläche senkrecht zu den Hauptachsen L der Gegenstände (10) folgt und sicherstellt, dass die Anordnung der Gruppe (20) von Gegenständen (10) als Bündel bei der Handhabung im Verfahren erhalten bleibt,
**dass** die Verfahrensschrittfolge b) mit der Gruppe (20) von Gegenständen (10) als Bündel einschließlich ihrer Umhüllung (30) durchgeführt wird,
**dass** nach dem Einbringen der Gruppe (20) von Gegenständen (10) in das Verpackungsmittel (40) das Verpackungsmittel (40) verschlossen wird und
**dass** vor dem Verschließen des Verpackungsmittels (40) die Umhüllung (30) entfernt wird oder die Umhüllung (30) im Verpackungsmittel (40) verbleibt.

2. Verfahren nach Anspruch 1, **dadurch gekennzeichnet,**
**dass** als Umhüllung (30) eine gut schließende Banderole verwendet wird.

3. Verfahren nach einem der vorangehenden Ansprüche, **dadurch gekennzeichnet,**
**dass** die Umhüllung (30) eine Greifvorrichtung aufweist.

4. Verfahren nach einem der vorangehenden Ansprüche, **dadurch gekennzeichnet,**
**dass** die Gegenstände (10) räumlich ineinander greifend oder in mindestens einem Querschnitt formschlüssig in einer Gruppe (20) angeordnet werden.

5. Verfahren nach einem der vorangehenden Ansprüche, **dadurch gekennzeichnet,**
**dass** mehrere mit jeweils einer Umhüllung (30) als Bündel vorfixierte Gruppen (20) von Gegenständen (10) mit Hilfe eines Sterilisationskorbs in den Sterilisationsraum eingebracht werden.

6. Verfahren nach einem der vorangehenden Ansprüche, **dadurch gekennzeichnet,**
**dass** die Gegenstände (10) eine individuelle Verpackung umfassen, die vorzugsweise eine Blisterverpackung ist.

7. Verfahren nach einem der vorangehenden Ansprüche, **dadurch gekennzeichnet,**
**dass** es sich bei den Gegenständen (10) um Einwegspritzen handelt.

8. Verfahren nach einem der vorangehenden Ansprüche, **dadurch gekennzeichnet,**
**dass** in ein Verpackungsmittel (40) genau zwei Gruppen (20) von Gegenständen (10) eingebracht werden.

9. Verfahren nach einem der vorangehenden Ansprüche, **dadurch gekennzeichnet,**
**dass** eine Umhüllung (30) auf polyolefinem Kunststoff verwendet wird.

10. Verfahren nach einem der vorangehenden Ansprüche, **dadurch gekennzeichnet,**
**dass** eine Umhüllung verwendet wird, die einen Indikator aufweist, der eine erfolgreiche Sterilisation anzeigt.

## Claims

1. A method for sterilization, preferably by means of a steam/air mixture, of objects (10) being equal to each other and being elongated, each comprising an axis L, which is the longest of the main axes of the object (10), and being twice as long as the other main axes of the object (10),
the method comprising the following steps:
a) the objects (10) are arranged in at least one group (20) such that their main axes L are aligned parallel to each other, and the objects (10) of the group (20) form a bundle,
b) the group (20) of objects (10) foamed in this manner is brought into a sterilization chamber, sterilized in said sterilization chamber, removed from said sterilization chamber, and placed into a packaging means (40) suitable for the complete envelopment of at least this group (20) of objects (10),
**characterized in that**
the group (20) of objects (10) is equipped with a casing (30) covering the same partially, however not completely, which is created by the bundle of objects (10), essentially follows the edge of a surface perpendicular to the main axes L of the objects (10), and ensures that the arrangement of the group (20) of objects (10) remains intact as a bundle during handling in the method,
the process step sequence b) is carried out using the group (20) of objects (10) as a bundle, including the casing (30) thereof,
after placing the group (20) of objects (10) into the packaging means (40) the packaging means (40) is closed, and
before the packaging means (40) is closed the casing (30) is removed, or the casing (30) remains within the packaging means (40).

2. The method according to claim 1, characterized that a well closing package band is utilized as the casing (30).

3. The method according to one of the previous claims, **characterized in that** the casing (30) comprises a gripping device.

4. The method according to one of the previous claims, **characterized in that** the objects (10) are arranged in a manner spatially engaging each other, in a form fitting manner in at least one cross-section in a group (20).

5. The method according to one of the previous claims, **characterized in that** multiple, previously fixed groups (20) of objects (10) each comprising one casing (30) are placed into the sterilization chamber by means of a sterilization basket.

6. The method according to one of the previous claims, **characterized in that** the objects (10) comprise an individual packaging, preferably being a blister packaging.

7. The method according to one of the previous claims, **characterized in that** the objects (10) are disposable syringes.

8. The method according to one of the previous claims, **characterized in that** exactly two groups (20) of objects (10) are placed into one packaging means (40).

9. The method according to one of the previous claims, **characterized in that** a casing (30) made of polyolefin plastic is utilized.

10. The method according to one of the previous claims, **characterized in that** a casing is utilized, having an indicator displaying successful sterilization.

## Revendications

1. Procédé pour la stérilisation, de préférence à l'aide d'un mélange vapeur-air, d'objets (10), qui sont de même nature entre eux et de forme allongée et présentent chacun un axe L, qui est le plus long des axes principaux de l'objet (10) et fait plus de deux fois la longueur des autres axes principaux de l'objet (10),
le procédé comprenant les étapes suivantes:
a) les objets (10) sont disposés en au moins un groupe (20), de telle sorte que leurs axes principaux L sont orientés parallèlement entre eux et les objets (10) du groupe (20) forment un paquet,
b) le groupe (20) ainsi formé d'objets (10) est introduit sous forme de paquet dans un espace de stérilisation, stérilisé dans l'espace de stérilisation, prélevé de l'espace de stérilisation et introduit dans un emballage (40), qui convient pour l'enveloppement complet d'au moins ce groupe (20) d'objets (10),
**caractérisé:**
**en ce que** le groupe (20) d'objets (10) est doté d'une enveloppe (30) le recouvrant partiellement, mais pas complètement, qui crée le paquet d'objets (10), suit essentiellement le bord d'une surface perpendiculairement aux axes principaux L des objets (10) et garantit que l'agencement du groupe (20) d'objets (10) est conservé comme paquet lors de la manipulation dans le procédé,
**en ce que** la succession d'étapes du procédé b) est mise en oeuvre avec le groupe (20) d'objets (10) sous forme de paquet, y compris son enveloppe (30),
**en ce que**, après l'introduction du groupe (20) d'objets (10) dans l'emballage (40), l'emballage (40) est fermé, et
**en ce que**, avant la fermeture de l'emballage (40), l'enveloppe (30) est enlevée ou bien l'enveloppe (30) reste dans l'emballage (40).

2. Procédé selon la revendication 1, **caractérisé:**
**en ce qu'**une bandelette fermant bien est utilisée comme enveloppe (30).

3. Procédé selon l'une des revendications précédentes, **caractérisé:**
**en ce que** l'enveloppe (30) présente un dispositif de préhension.

4. Procédé selon l'une des revendications précédentes, **caractérisé:**
**en ce que** les objets (10) sont disposés en s'imbriquant les uns dans les autres dans l'espace ou par complémentarité de formes dans au moins une section dans un groupe (20).

5. Procédé selon l'une des revendications précédentes, **caractérisé:**
**en ce que** plusieurs groupes (20) d'objets (10), préfixés avec respectivement une enveloppe (30) comme paquet, sont introduits à l'aide d'un panier de stérilisation dans l'espace de stérilisation.

6. Procédé selon l'une des revendications précèdent **caractérisé:**
**en ce que** les objets (10) comprennent un emballage individuel qui forme de préférence un emballage blister.

7. Procédé selon l'une des revendications précédentes, **caractérisé en ce que**:
en ce qui conceme les objets (10), il s'agit de seringues à usage unique.

8. Procédé selon l'une des revendications précédentes, **caractérisé:**
**en ce qu'**exactement deux groupes (20) d'objets sont introduits dans un emballage (40).

9. Procédé selon l'une des revendications précédentes, **caractérisé:**
**en ce qu'**une enveloppe (30) à base de plastique polyoléfinique est utilisée.

10. Procédé selon l'une des revendications précédentes, **caractérisé:**
**en ce qu'**on utilise une enveloppe qui présente un indicateur, lequel indique une stérilisation réussie
